# EUROPEAN PATENT SPECIFICATION

(11) **EP 2 135 622 B1**
(45) Date of publication and mention of the grant of the patent: **02.05.2012**
(21) Application number: 09250632.8
(22) Date of filing: 05.03.2009
(51) Int. Cl.: A61K 45/06, A61K 31/132, A61K 33/00

(54) **Compositions for providing an analgesic effect to the skin**
Zusammensetzungen zur Ausübung eines schmerzlindernden Effekts auf die Haut
Compositions pour la fourniture d'un effet analgésique sur la peau

(30) Priority: 06.03.2008 US 43373
(43) Date of publication of application: 23.12.2009
(73) Proprietor: Johnson & Johnson Consumer Companies, Inc., Skillman, NJ 08558 (US)
(72) Inventor: Liebel, Frank T., Point Pleasant, NJ 08742 (US); Southall, Michael David, Lawrenceville, NJ 08648 (US)
(74) Representative: Kirsch, Susan Edith

(56) References cited:
- US-A1- 2006 193 815
- TAKANO NORIKAZU; ARAI IWAO; HASHIMOTO YUKI; KURACHI MICHIO: "Evaluation of antipruritic effects of several agents on scratching behavior by NC/Nga mice" EUROPEAN JOURNAL OF PHARMACOLOGY, vol. 495, no. 2-3, 14 July 2004 (2004-07-14), pages 159-165, XP002578696 ISSN: 0014-2999
- TAKANO NORIKAZU; ARAI IWAO; KURACHI MICHIO: "Possible antipruritic effects of K+ channel openers in mice" JOURNAL OF DERMATOLOGICAL SCIENCE, vol. 35, no. 1, June 2004 (2004-06), pages 71-73, XP002578697 ISSN: 0923-1811
- LUCKY A W; PIACQUADIO D J; DITRE C M; DUNLAP F; KANTOR I; PANDYA A G; SAVIN R C; THARP M D: "A randomized, placebo-controlled trial of 5% and 2% topical minoxidil solutions in the treatment of female pattern hair loss." JOURNAL OF THE AMERICAN ACADEMY OF DERMATOLOGY APR 2004 LNKD- PUBMED:15034503, vol. 50, no. 4, April 2004 (2004-04), pages 541-553, XP002578698 ISSN: 0190-9622

## Description

### Field of the Invention

The present invention relates to compositions containing an amine-containing compound and a sensation-blocking agent selected from the group consisting of a potassium salt and a potassium channel agonist, which compositions provide an analgesic effect to the skin when applied to an area of skin exhibiting symptoms of itch, pain, stinging, burning sensations, and the like.

### Background of the Invention

Eczema, psoriasis, acne, rosacea, contact irritant dermatitis, atopic dermatitis, allergic dermatitis, sunlight-induced dermatoses and dry skin are all conditions that cause itch, pain, stinging and/or burning sensations of the skin. Consumers have relied on analgesic agents to treat these conditions for many years. Analgesic agents reduce neurosensory sensations, such as pain, itch, sting and burning sensations, without resulting in loss of consciousness. Analgesics are sometimes referred to as painkiller medications. There are many different types of analgesic medications available in both prescription and over-the-counter preparations. Examples of analgesic drugs include aspirin, acetaminophen, ibuprofen, naproxen, the COX-2 inhibitor celecoxib, and narcotic drugs including morphine, oxycodone and hydrocodone.

Topical analgesic agents are also called counter-irritants. The name derives from the fact that these agents cause a reddening of the skin by causing the blood vessels of the skin to dilate, which gives a soothing feeling of warmth. The term counter-irritant refers to the idea that irritation of the sensory nerve endings alters or offsets pain in the underlying muscle or joints that are served by the same nerves. Examples of topical analgesic agents include capsaicin, capsicum oleoresin, choline salicylate, ethyl salicylate, glycol salicylate, methyl salicylate, menthol, salicylic acid and turpentine oil. Although analgesic agents are effective in relieving irritation, some have undesiarble side effects. For example, some analgesics slow the heart rate of the consumer. Other analgesics are difficult to deliver topically due to skin permeability issues. There is a continuing need for compositions that provide an analgesic effect to the skin without the need for the use of conventional analgesic materials, thereby relieving or reducing itch, pain, stinging and/or burning sensations of the skin while avoiding the need to use conventional analgesic materials.

### Summary of the Invention

The present invention provides a composition including an amine-containing compound selected from the group consisting of Formula I and Formula II 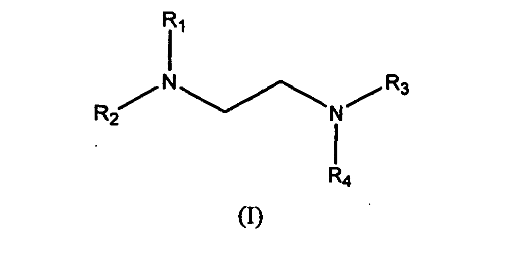 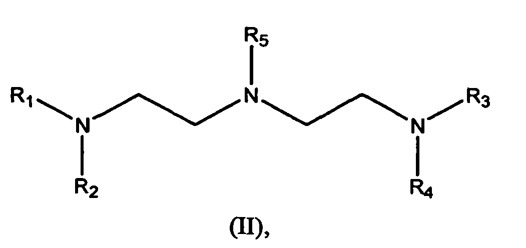 wherein R₁, R₂, R₃, R₄ and R₅ independently are selected from the group consisting of hydrogen, C₁-C₆ alkyl and C₁-C₆ hydroxyalkyl; or a cosmetically-acceptable salt thereof; and a sensation-blocking agent selected from the group consisting of a potassium salt and a potassium channel agonist, wherein the amine-containing compound and the sensation-blocking agent are present in amounts effective to provide an analgesic effect to the skin when applied to an area of skin exhibiting symptoms of itch, pain, stinging, burning sensations, and the like, without the presence of conventional analgesic materials.

### Detailed Description of the Invention

The compositions of the present invention include an amine-containing compound selected from the group consisting of Formula I and Formula II, 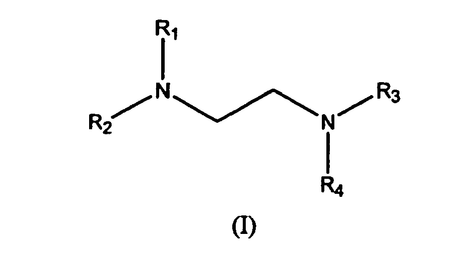 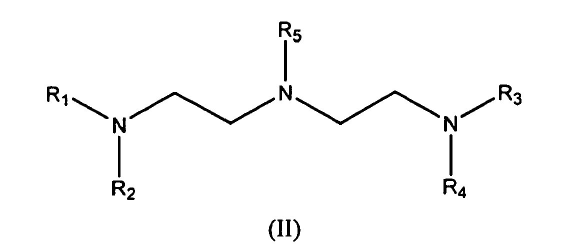 wherein R₁, R₂, R₃, R₄ and R₅ independently are selected from the group consisting of hydrogen, C₁-C₆ alkyl, and C₁-C₆ hydroxyalkyl; or a cosmetically-acceptable salt thereof. In one embodiment, the compositions of the present invention contain at least one compound of Formula I where R₁, R₂, R₃ and R₄ each are selected from the group consisting of C₁-C₃ alkyl and C₁-C₃ alkanol. In a further embodiment, at least one of R₁, R₂, R₃ and R₄ of Formula I is a C₂-C₃ alkanol group bearing at least one hydroxyl group. Examples of compounds of Formula I include, but are not limited to, N,N,N',N'-Tetrakis(2-hydroxypropyl)ethylenediamine (THPED), N, N, N', N'-Tetrakis (2-hydroxyethyl) ethylene diamine (THEED), N, N, N', N'-tetramethylethylene diamine (TEMED), the structures of which are set forth below, enantiomers thereof, or diastereoisomers thereof.

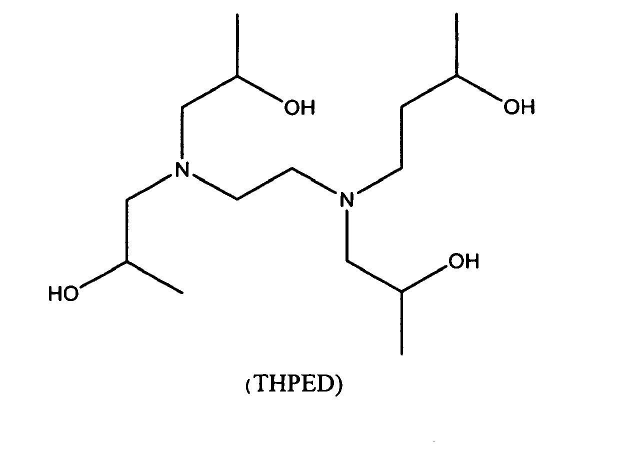

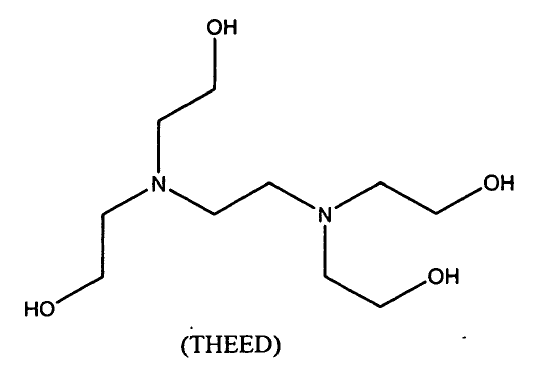

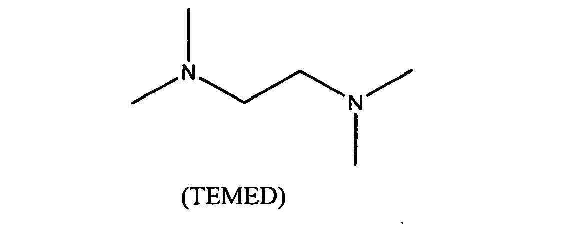

Examples of compounds of Formula II include, but are not limited to, Tris[2-(isopropylamino)ethyl]amine, the structure of which is set forth below, enantiomers thereof, or diastereoisomers thereof.

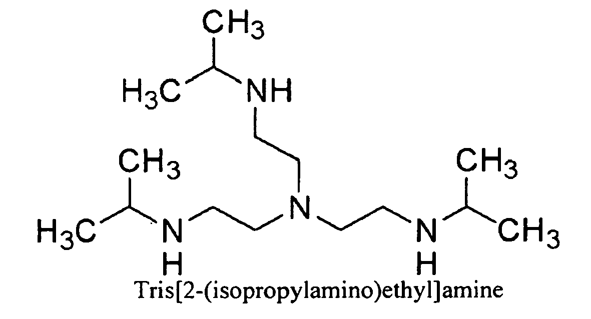

The synthesis ofN,N,N',N'-tetrakis (2-hydroxypropyl) ethylenediamine from the reaction of ethylenediamine with propylene oxide is described in U.S. Patent No. 2,697,118.

The amine-containing compounds of the present invention may also be present in the form of cosmetically-acceptable salts. For use in medicine, the salts of the compounds of this invention refer to non-toxic "cosmetically-acceptable salts," or cosmetically-acceptable acidic/anionic or basic/cationic salts. Cosmetically-acceptable acidic/anionic salts include, but are not limited to, acetate, benzenesulfonate, benzoate, bicarbonate, bitartrate, bromide, calcium edetate, camsylate, carbonate, chloride, citrate, dihydrochloride, edetate, edisylate, estolate, esylate, fumarate, glyceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, methylsulfate, mucate, napsylate, nitrate, pamoate, pantothenate, phosphate/diphospate, polygalacturonate, salicylate, stearate, subacetate, succinate, sulfate, tannate, tartrate, teoclate, tosylate and triethiodide. Cosmetically-acceptable basic/cationic salts include, but are not limited to, salts of aluminum, benzathine, calcium, chloroprocaine, choline, diethanolamine, ethylenediamine, lithium, magnesium, meglumine, potassium, procaine, sodium and zinc. Other salts may, however, be useful in the preparation of compositions according to the present invention. Organic or inorganic acids also include, but are not limited to, hydriodic, perchloric, sulfuric, phosphoric, propionic, glycolic, methanesulfonic, hydroxyethanesulfonic, oxalic, 2-naphthalenesulfonic, p-toluenesulfonic, cyclohexanesulfamic, saccharinic or trifluoroacetic acid.

The amine-containing compound is present in the composition at an amount of from about 0.75% to about 10%, for example 0.75% to 2%, by weight of the composition.

Preferably the amine-containing compound is selected from the group consisting of N,N,N',N'-Tetrakis(2-hydroxypropyl)ethylenediamine and Tris[2-(isopropylamino)ethyl]amine. Most preferably the amine-containing compound is N,N,N',N'-Tetrakis(2-hydroxypropyl)ethylenediamine.

The compositions of the present invention also contain a sensation-blocking agent selected from the group consisting of a potassium salt and a potassium channel agonist. By sensation-blocking agent, it is meant that such agents, when applied in combination with the amine-containing compound to an area of skin exhibiting at least one symptom of itch, pain, stinging, burning sensations, and the like, provide potassium ion at the symptomatic area of skin to which the composition is administered, so as to provide an analgesic effect, as described herein.

The potassium ion may be delivered directly to the area of the skin by topical administration of a composition containing potassium ion therein, or by topical administration of a composition containing a sensation-blocking agent that provides for generation of the potassium ion at the area of skin to which the composition is administered. Such agents may be selected from the group consisting of potassium salts and potassium channel agonists. Potassium salts may be used to deliver potassium ion to the site of the skin exhibiting symptoms, while potassium channel agonists permit potassium ions to be generated at the site of the skin exhibiting symptoms.

Compositions of the present invention contain the sensation-blocking agents selected from the group consisting of a potassium salt and a potassium channel agonist in amounts effective to provide an analgesic effect to the skin without the requirement of a conventional analgesic material. In determining such analgesic effect to the skin, known methods as set forth in Liebel et al., Arch Dermatol Res. 298:191-199, 2006, were employed. In particular, percent inhibition of scratching and percent inhibition of ear edema were examined as described in the examples herein. Compositions of the present invention are effective in providing at least about 25 percent inhibition of ear edema, or at least about 30 percent. In certain embodiments, the compositions provide greater than about 40 percent inhibition of ear edema. In addition, certain embodiments of the present invention provide greater than about 50 percent, or greater than about 60 percent inhibition of scratching, according to test methods utilized.

In the case of potassium salts, the amount of the potassium salt in the compositions of the present invention is effective to provide potassium ion in the composition at a range of from about 0.15% to about 10%, for example from about 0.20% to about 2%, by weight of the composition. Suitable potassium salts include, but are not limited to, potassium lactate, potassium bromide, potassium carbonate, potassium acetate, potassium chlorate, potassium chloride, potassium chromate, potassium cyanide, potassium dichromate, potassium iodide, potassium nitrate, potassium sulfate, potassium pyrophosphate, potassium fluorosilicate and potassium sodium tartrate. Preferably the potassium salt is selected from potassium lactate, potassium carbonate, potassium acetate and potassium chloride. Most preferably the potassium salt is selected from potassium lactate and potassium chloride.

Suitable potassium channel agonist include, without limitation, a chemically diverse group of agents such as minoxidil, pinacidil, diazoxide, cromakalin, nicorandil, and aprikalim. (See Lawson., 2000; Takano et. al., 2004, and those listed in table 4.3 of Biochemical Targets of Plant Bioactive Compounds, Polya., 2003). Preferably the potassium salt is minoxidil. Compositions of the present invention may comprise from about 0.25% to about 10% percent by weight of the potassium channel agonist, or from about 0.25% to about 2% by weight of the composition. While not intending to be limited by the following, potassium channel agonists induce an efflux of potassium from the cells. This creates a hyperpolarized state that restricts calcium from entering back into the cells and dampens cellular excitability. Potassium channel agonists also affect neurons by decreasing neuronal excitability and interfering with neurotransmission. Itch, pain, sting and burning sensations are transmitted by sensory neurons such as unmyelinated C-fibers. Thus, the effects of potassium channel agonists on neurotransmission inhibit these neurosensory sensations.

A suitable composition comprises from about 0.75 percent to about 10 percent by weight of the amine-containing compound and from about 0.25 percent to about 10 percent by weight of the sensation-blocking agent selected from the group consisting of a potassium salt and a potassium channel agonist.

In a suitable composition wherein the sensation-blocking agent is a potassium salt, the amount of amine-containing compound ranges from about 0.75 percent to about 10 percent by weight of the composition and the amount of the potassium salt is effective to provide potassium ion in the composition at a range from about 0.15 percent to about 10 percent by weight of the composition.

In another suitable composition wherein the sensation-blocking agent is a potassium salt, the amount of amine-containing compound ranges from about 0.75 percent to about 2 percent by weight of the composition and the amount of potassium salt is effective to provide potassium ion in the composition at a range from about 0.75 percent to about 2 percent by weight of the composition.

In a further suitable composition wherein the sensation-blocking agent is a potassium channel agonist, the amount of amine-containing compound ranges from about 0.75 percent to about 10 percent by weight of the composition and the amount of the potassium channel agonist ranges from about 0.25 percent to about 10 percent by weight of the composition.

The compositions of the present invention are prepared by standard techniques well known to those skilled in the art. If the composition comprises more than one phase, in general the different phases will be prepared separately, with materials of similar phase partitioning being added in any order. The two phases will then be combined with vigorous stirring to form the multiphase system, e.g., an emulsion or dispersion. Any ingredients in the formulation with high volatility, or which are susceptible to hydrolysis at high temperatures, will usually be added post-mixing of the different phases with gentle stirring.

The compositions of the present invention are useful for relieving or reducing symptoms of itch, pain, stinging and/or burning sensations of the skin by providing an analgesic effect. By analgesic effect, it is meant that the composition, when applied to an area of skin suffering from at least one symptom of itch, pain, stinging or burning sensations, provides relief or reduction of such symptoms that are similar to relief or reduction of such symptoms provided by known analgesic compositions, but without the negative side affects of such known analgesic compositions.

The compositions are topically applied to skin exhibiting these symptoms. As used herein, "topically applying" means directly laying on or spreading on outer skin, e.g., by use of the hands or an applicator such as a wipe, puff, roller or spray. As used herein, "cosmetically-acceptable" means that the product(s), compound(s), or composition(s) which the term describes are suitable for use in contact with tissue of a mammal (e.g., the skin or hair) without undue toxicity, incompatibility, instability, irritation, allergic response, and the like. This term is not intended to limit the compound/product/composition to which it describes for use solely as a cosmetic, e.g., the ingredient/product may be used as a pharmaceutical. As used herein, "topical carrier" means one or more compatible solid or liquid filler diluents that are suitable for topical administration to a mammal. Examples of topical carriers include, but are not limited to, water, waxes, oils, emollients, emulsifiers, thickening agents, gelling agents, and mixtures thereof.

The compositions of the present invention may be provided as formulations suitable for topical application to skin. The composition may comprise a cosmetically-acceptable topical carrier. The cosmetically-acceptable topical carrier may comprise from about 50% to about 99.99%, by weight, of the composition, e.g., from about 80% to about 95%, by weight, of the composition. The compositions may be made into a wide variety of product types that include, but are not limited to, solid and liquid compositions, such as lotions, creams, gels, sticks, sprays, shaving creams, ointments, cleansing liquid washes and solid bars, shampoos, pastes, powders, mousses, shaving creams and wipes. These product types may comprise several types of cosmetically acceptable topical carriers including, but not limited to, solutions, emulsions, e.g., microemulsions and nanoemulsions, gels, solids and liposomes. The following are non-limitative examples of such carriers. Other carriers can be formulated by those of ordinary skill in the art.

The compositions of the present invention can be formulated as solutions. Solutions typically include an aqueous solvent, e.g., from about 50% to about 99.99%, or from about 90% to about 99%, of a cosmetically-acceptable aqueous solvent. Topical compositions of the subject invention may be formulated as a solution comprising an emollient. Such compositions preferably contain from about 2% to about 50% of an emollient(s). As used herein, "emollients" refer to materials used for the prevention or relief of dryness, as well as for the protection of the skin. A wide variety of suitable emollients are known and may be used herein. See International Cosmetic Ingredient Dictionary and Handbook, eds. Wenninger and McEwen, pp. 1656-61, 1626, and 1654-55 (The Cosmetic, Toiletry, and Fragrance Assoc., Washington, D.C., 7th Edition, 1997) (hereinafter "INCI Handbook").

A lotion can be made from such a solution. Lotions typically comprise from about 1% to about 20%, e.g., from about 5% to about 10%, of an emollient(s) and from about 50% to about 90%, e.g., from about 60% to about 80%, of water.

Another type of product that may be formulated from a solution is a cream. A cream typically comprises from about 5% to about 50%, e.g., from about 10% to about 20%, of an emollient(s) and from about 45% to about 85%, e.g., from about 50% to about 75%, of water.

Yet another type of product that may be formulated from a solution is an ointment. An ointment may comprise a simple base of animal or vegetable oils or semisolid hydrocarbons. An ointment may comprise from about 2% to about 10% of an emollient(s) plus from about 0.1% to about 2% of a thickening agent(s). A more complete disclosure of thickening agents or viscosity increasing agents useful herein can be found in the INCI Handbook pp. 1693-1697.

The compositions of the present invention may be formulated as emulsions. If the carrier is an emulsion, from about 1% to about 10%, e.g., from about 2% to about 5%, of the carrier comprises an emulsifier(s). Emulsifiers may be nonionic, anionic or cationic. Suitable emulsifiers are disclosed in, for example, INCI Handbook, pp.1673-1686.

Lotions and creams can be formulated as emulsions. Typically such lotions comprise from 0.5% to about 5% of an emulsifier(s). Such creams would typically comprise from about 1% to about 20%, e.g., from about 5% to about 10% of an emollient(s); from about 20% to about 80%, e.g., from 30% to about 70%, of water; and from about 1% to about 10%, e.g., from about 2% to about 5%, of an emulsifier(s).

Single emulsion skin care preparations of the oil-in-water type and water-in-oil type, such as lotions and creams, are well-known in the cosmetic art and are useful in the subject invention. Multiphase emulsion compositions, such as the water-in-oil-in-water type are also useful in the subject invention. In general, such single or multiphase emulsions contain water, emollients, and emulsifiers as essential ingredients.

The compositions of this invention can also be formulated as a gel, e.g., an aqueous gel using a suitable gelling agent(s). Suitable gelling agents for aqueous gels include, but are not limited to, natural gums, acrylic acid and acrylate polymers and copolymers, and cellulose derivatives, e.g., hydroxymethyl cellulose and hydroxypropyl cellulose. Suitable gelling agents for oils such as mineral oil include, but are not limited to, hydrogenated butylene/ethylene/styrene copolymer and hydrogenated ethylene/propylene/styrene copolymer. Such gels typically comprise between about 0.1% and 5%, by weight, of such gelling agents.

The compositions of the present invention can also be formulated into a solid formulation, e.g., a wax-based stick, soap bar composition, powder, or a wipe containing powder.

The compositions of the invention may contain, in addition to the aforementioned components, a wide variety of additional oil-soluble materials and/or water-soluble materials conventionally used in compositions for use on skin, hair, and nails at their art-established levels.

The compositions may be applied one or more times a day, for example twice a day. The amount used will vary with the age and physical condition of the end user, the duration of the treatment, the specific compound, product, or composition employed, the particular cosmetically-acceptable carrier utilized, and like factors. Several examples are described below. The invention should not be construed to be limited to the details thereof.

### Examples

In the following examples, the tables provide "Topical Dose, as % w/v" for "Potassium Ion (delivered as 1% Potassium salt)", which refers to % w/v for the Potassium Ion itself.

Compound 48/80 is commercially available from Sigma-Aldrich and is the condensation product of N-methyl-p-methoxyphenethylamine with formaldehyde.

### Example 1 - Scratching against Compound 48/80

Albino male CD-1 mice, 7-9 weeks old, were used. Testing was based on known methods (See Liebel et al.,Arch Dermatol Res. 298:191-199, 2006.) Test agents were prepared at concentrations noted in Table 1 in 100% ethanol and 50µL was applied topically to the shaved dorsal skin 30 minutes prior to induction of scratching (8 mice per treatment group). Compound 48/80 (50µg/50µL: w/v) was then injected inter-dermal to the dorsal area and scratches were then counted for 30 minutes. The results are shown in Table 1.

**Table 1**

| Treatment | Topical Dose, as % w/v | % Inhibition of Scratching* |
|---|---|---|
| Tetrahydroxypropyl ethylenediamine | 1 | 9.39 |
| Potassium Ion (delivered as 1% Potassium Lactate) | 0.206 | -8.84 |
| Potassium Ion (delivered as 1% Potassium Lactate) + Tetrahydroxypropyl ethylenediamine | 0.206 +1 | 61.18 |

| | | |
|---|---|---|
| * % Inhibition = ((Untreated Group Number of Scratches - Treatment Group Number of Scratches) / Untreated Group Number of Scratches) x 100 | | |

The combination of potassium lactate and tetrahydroxypropyl ethylenediamine was highly effective in this model. In comparison, equal levels of potassium ion or tetrahydroxypropyl ethylenediamine alone exhibited very weak to no inhibition. These results demonstrate that the combination of appropriate amounts of potassium ion, as delivered by potassium lactate, and tetrahydroxypropyl ethylenediamine synergistically produced an analgesic effect in reducing scratching, and would therefore be expected to prevent and/or treat itch, pain, sting and/or burning sensations.

### Example 2

Neurosensory inflammation, also referred to as neurogenic inflammation, is a type of inflammation triggered by sensory nerve activation in skin. Certain natural substances that act on vanilloid receptors cause sensory nerves (C-fibers) to release inflammatory neuropeptides. In mouse skin, an edema response occurs rapidly upon application of a vanilloid receptor activator, such as capsaicin or resiniferatoxin (RTX). Compounds that inhibit the response to neurosensory stimulation could be useful as topical analgesics, itch or sting inhibitors or soothing agents for irritated skin.

Albino male CD-1 mice, 7-9 weeks old, were used. Induction of neurogenic inflammation in the mouse ear was based on known methods (See Liebel et al., Arch Dermatol Res. 298:191-199, 2006.). A 20-µL volume of Resiniferatoxin (0.05%) prepared in acetone was applied to the left ears (7 mice per treatment group). The right ear was not treated. Test agents were prepared in a 70% ethanol/30% propylene glycol vehicle and applied to the left ear (20 µL) immediately after resiniferatoxin challenge. The percent inhibition was calculated by comparing treatments to resiniferatoxin alone. Anti-neurogenic inflammation effects of compounds are evident as an inhibition of the increase in ear weight. The results are shown in Table 2.

**Table 2**

| Treatment | Topical Dose, as % w/v | % Inhibition of the Ear Edema* |
|---|---|---|
| Lidocaine | 0.5 | 28.7 |
| Tetrahydroxypropyl ethylenediamine | 1 | 4.04 |
| Tris[2-(isopropylamino) ethyl]amine | 0.5 | 13.62 |
| Potassium Ion (delivered as 1% Potassium Lactate) | 0.206 | 13.52 |
| Potassium Ion (delivered as 1% Potassium Lactate) + Tetrahydroxypropyl ethylenediamine | 0.206 +1 | 43.83 |
| Potassium Ion (delivered as 1% Potassium Lactate) + Tris[2-(isopropylamino) ethyl]amine | 0.206 + 0.5 | 48.83 |

| | | |
|---|---|---|
| * % Inhibition = ((Untreated Biopsy Weight - Treatment Biopsy Weight) / Untreated Biopsy Weight) x 100 | | |

The use of potassium lactate, or tetrahydroxypropyl ethylenediamine or Tris[2-(isopropylamino)ethyl]amine, each one by itself, failed to significantly inhibit the neurogeniic inflammatory response to resiniferatoxin. However, the combination of potassium lactate and tetrahydroxypropyl ethylenediamine or Tris[2-(isopropylamino) ethyl]amine significantly inhibited the neurogenic inflammatory response to resiniferatoxin. The combination of potassium lactate and tetrahydroxypropyl ethylenediamine or Tris[2-(isopropylamino)ethyl]amine was highly effective in this model. In comparison, while lidocaine, a known anesthetic compound (Morgan M and Russell WJ. Br J Anaesth. 1975 47:586-591, 1975), was also active, the combination of the present invention was significantly more effective than 0.5% lidocaine alone. Together these results demonstrate that the combination of potassium ion, as delivered by potassium lactate in the specific example, and tetrahydroxypropyl ethylenediamine or Tris[2-(isopropylamino)ethyl]amine produced an analgesic effect in reducing neurogenic inflammation, and would therefore be expected to prevent and/or treat itch, pain, sting and/or burning sensations.

### Example 3

Different ions of chloride were evaluated in the resiniferatoxin neurogenic inflammation model in mice as described in Example 2. The results are shown in Table 3.

**Table 3**

| Treatment | Topical Dose, as % w/v | % Inhibition of the Ear Edema* |
|---|---|---|
| Potassium Ion (delivered as 1% Potassium Chloride) | 0.524 | 9.98 |
| Potassium Ion (delivered as I % Potassium Chloride) + Tetrahydroxypropyl ethylenediamine | 0.524 +1 | 45.89 |
| Sodium Chloride | 1 | 7.06 |
| Sodium Chloride + Tetrahydroxypropyl ethylenediamine | 1+1 | 6.01 |
| Calcium Chloride | 1 | -3.24 |
| Calcium Chloride + Tetrahydroxypropyl ethylenediamine | 1+1 | 15.17 |

| | | |
|---|---|---|
| * % Inhibition = ((Untreated Biopsy Weight - Treatment Biopsy Weight) / Untreated Biopsy Weight) x 100 | | |

The combination of potassium chloride and tetrahydroxypropyl ethylenediamine was highly effective in this model. In comparison, sodium chloride and calcium chloride in combination with tetrahydroxypropyl ethylenediamine exhibited very weak inhibition of the neurogenic inflammation.

### Example 4

Different potassium salts were studied in the Resiniferatoxin neurogenic inflammation model in mice as described in Example 2. The results are shown in Table 4.

**Table 4**

| Treatment | Topical Dose, as % w/v | % Inhibition of the Ear Edema* |
|---|---|---|
| Potassium Ion (delivered as 1% Potassium Acetate) | 0.398 | -2.89 |
| Potassium Ion (delivered as 1% Potassium Acetate) + Tetrahydroxypropyl ethylenediamine | 0.398 +1 | 33.30 |
| Potassium Ion (delivered as I % Potassium Carbonate) | 0.283 | 4.52 |
| Potassium Carbonate + Tetrahydroxypropyl ethylenediamine | 0.283 +1 | 33.55 |
| Potassium Ion (delivered as 1% Potassium Chloride) | 0.524 | 9.98 |
| Potassium Ion (delivered as 1% Potassium Chloride) + Tetrahydroxypropyl ethylenediamine | 0.524+1 | 45.89 |
| Potassium Lactate | 0.206 | 13.52 |
| Potassium Lactate + Tetrahydroxypropyl ethylenediamine | 0.206 +1 | 43.83 |

| | | |
|---|---|---|
| * % Inhibition = ((Untreated Biopsy Weight - Treatment Biopsy Weight) / Untreated Biopsy Weight) x 100 | | |

The combination of tetrahydroxypropyl ethylenediamine with any of the potassium salts tested provided an inhibition against induced neurogenic inflammation. The potassium salts alone had very weak to no activity against the neurogenic inflammation. Together, these results demonstrate that various potassium salts in combination with tetrahydroxypropyl ethylenediamine produced an analgesic effect in reducing neurogenic inflammation, and would therefore be expected to prevent and/or treat itch, pain, sting and/or burning sensations.

### Example 5

A topical formulation containing potassium lactate and tetrahydroxypropyl ethylenediamine was created for testing following the ingredient list of Table 5.

**Table 5**

| Ingredient Trade Name | Formula % Weight |
|---|---|
| Purified Water | 68.5 |
| EleFac I-205 | 6 |
| DMS Concentrate Probiol N03031 (Kuhs Gmbh) | 3.8 |
| Glycerin | 3 |
| DC 9566 Silicone Elastomer | 3 |
| Hexylene Glycol | 2.5 |
| D-Panthenol, USP | 2 |
| Oliwax | 2 |
| Betafin BP-20 | 1.5 |
| Advanced Moisture Complex | 1.5 |
| PURASAL HiPure P (Potassium Lactate) | 1 |
| DC 200 Fluid 50 cst | 1 |
| Crodacol C-95 | 1 |
| Tetrahydroxypropyl ethylenediamine (Neutrol TE) | 1 |
| Carbopol Ultrez 10 | 0.75 |
| Sodium Hydroxide, 20% | 0.7 |
| Nipagin M | 0.3 |
| Versene NA | 0.2 |
| Keltrol 1000 | 0.15 |
| Nipasol M | 0.1 |
| | 100 |

### Example 6

The formulation prepared in Example 5 was tested in the Resiniferatoxin neurogenic inflammation model in mice as described in Example 2. The results are shown in Table 6.

**Table 6**

| Treatment | Topical Dose, as % w/v | % Inhibition of the Ear Edema* |
|---|---|---|
| Placebo Formulation | 0 | 1.22 |
| Potassium Ion (delivered as 1% Potassium Lactate) + Tetrahydroxypropyl ethylenediamine Formulation | 0.206 +1 | 30.18 |

| | | |
|---|---|---|
| * % Inhibition = ((Untreated Biopsy Weight - Treatment Biopsy Weight) / Untreated Biopsy Weight) x 100 | | |

These results demonstrate that potassium lactate in combination with tetrahydroxypropyl ethylenediamine produced an analgesic effect in reducing neurogenic inflammation, and would therefore be expected to prevent and/or treat itch, pain, sting and/or burning sensations.

### Example 7

A blinded controlled clinical usage trial to evaluate the tolerance and efficacy of a formulation containing potassium lactate and tetrahydroxypropyl ethylenediamine was used on 30 healthy adult human subjects having mild to moderate atopic dermatitis as determined by the Hanifin and Rajka criteria. The subjects also suffered from itch associated with their atopic dermatitis. A clinical and subject self-assessment of the itch sensation was taken at baseline, one hour after application and 24 hours after a single application. The results are shown in Table 7.

**Table 7**

| Treatment | Time Point* | Clinical Assessment Score ** | Self-Assessment Score*** |
|---|---|---|---|
| Potassium Ion (delivered as 1% Potassium Lactate) + Tetrahydroxypropyl ethylenediamine Formulation | Baseline | 1.94 | 3.75 |
| | 1 hour after | 0.00 | - |
| | 24 hours after | 0.39 | 6.18 |

| | | | |
|---|---|---|---|
| * time after a single application of the formulation ** (0= No itch, 1= Mild itch, 2= Moderate itch, 3= Severe Itch) ** (10 point scale 1= very itchy, 10= does not itch at all) | | | |

A single application of a formulation containing potassium lactate and tetrahydroxypropyl ethylenediamine was able to deliver a prolonged reduction in the itch sensation in subjects with itchy skin due to atopic dermatitis. These results demonstrate that potassium lactate and tetrahydroxypropyl ethylenediamine produced an analgesic effect in reducing itch, and would therefore be expected to prevent and/or treat itch, pain, sting and/or burning sensations.

### Example 8: Potassium Channel Agonists against scratching by Compound 48/80

Albino male CD-1 mice, 7-9 weeks old, were used. Testing was based on known methods (See Liebel et al., Arch Dermatol Res. 298:191-199, 2006.) Test agents were prepared in 100% ethanol and 50uL was applied topically to the shaved dorsal skin 30 minutes prior to induction of scratching (8 mice per treatment group). Compound 48/80 (50ug/50uL w/v) was then injected inter-dermal to the dorsal area and scratches were then counted for 30 minutes.

**Table 8**

| Treatment | Topical Dose, as % w/v | % Inhibition of Scratching* |
|---|---|---|
| Tetrahydroxypropyl ethylenediamine | 1 | 9.39 |
| Minoxidil | 1 | 8.42 |
| Minoxidil + Tetrahydroxypropyl ethylenediamine | 1+1 | 51.74 |
| Minoxidil + Tetrahydroxypropyl ethylenediamine | 0.5+1 | 46.05 |
| Minoxidil + Tetrahydroxypropyl ethylenediamine | 0.25+1 | 1.80 |
| Pinacidil | 1 | 17.03 |
| Pinacidil + Tetrahydroxypropyl ethylenediamine | 1+1 | 80.44 |
| Pinacidil + Tetrahydroxypropyl ethylenediamine | 0.25+1 | 28.53 |

| | | |
|---|---|---|
| * % Inhibition = ((Untreated Group Number of Scratches - Treatment Group Number of Scratches) / Untreated Group Number of Scratches) x 100 | | |

These results demonstrate that potassium channel agonists in combination with tetrahydroxypropyl ethylenediamine produced a synergistic analgesic effect in reducing scratching, and would therefore be expected to prevent and/or treat itch, pain, sting and/or burning sensations.

## Claims

1. A composition, comprising:
an amine-containing compound selected from the group consisting of Formula I and Formula II 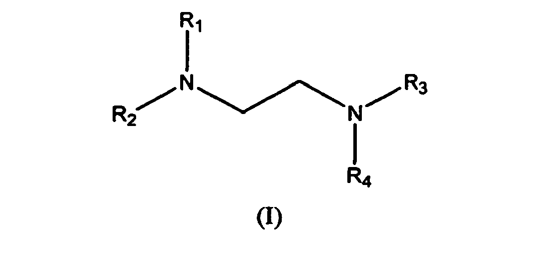 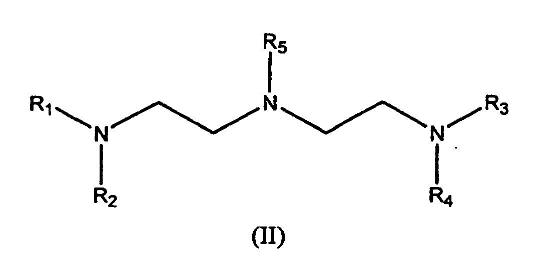 wherein R₁, R₂, R₃, R₄ and R₅ independently are selected from the group consisting of hydrogen, C₁-C₆ alkyl, and C₁-C₆ hydroxyalkyl; or a cosmetically-acceptable salt thereof; and
a sensation-blocking agent selected from the group consisting of a potassium salt and a potassium channel agonist,
wherein the amine-containing compound and the sensation-blocking agent are present in amounts effective to provide an analgesic effect to the skin when applied thereto.

2. The composition according to claim 1 wherein the amine-containing compound is selected from the group consisting of N,N,N',N'-Tetrakis(2-hydroxypropyl)ethylenediamine and Tris[2-(isopropylamino)ethyl]amine.

3. The composition according to claim 1 or claim 2 wherein the amount of amine-containing compound ranges from about 0.75 percent to about 10 percent by weight of the composition, preferably from about 0.75 percent to about 2 percent by weight of the composition.

4. The composition according to any one of claims 1 to 3 wherein the sensation-blocking agent is a potassium salt.

5. The composition according to claim 4 wherein the potassium salt is selected from the group consisting of potassium lactate, potassium bromide, potassium carbonate, potassium acetate, potassium chlorate, potassium chloride, potassium chromate, potassium cyanide, potassium dichromate, potassium iodide, potassium nitrate, potassium sulfate, potassium pyrophosphate, potassium fluorosilicate and potassium sodium tartrate.

6. The composition according to claim 4 or claim 5 wherein the amount of the potassium salt is effective to provide potassium ion in the composition at a range from about 0.15 percent to about 10 percent by weight of the composition, preferably from about 0.20 percent to about 2 percent by weight of the composition.

7. The composition according to any one of claims 1 to 3 wherein the sensation-blocking agent is a potassium channel agonist.

8. The composition according to claim 7 wherein the potassium channel agonist is selected from the group consisting of minoxidil, pinacidil, diazoxide, cromakalin, nicorandil and aprikalim.

9. The composition according to claim 7 or claim 8 wherein the amount of the potassium channel agonist ranges from about 0.25 percent to about 10 percent by weight of the composition, preferably from about 0.25 percent to about 2 percent by weight of the composition.

10. The composition according to any one of claims 1 to 9 comprising from about 0.75 percent to about 10 percent by weight of the amine-containing compound and from about 0.25 percent to about 10 percent by weight of the sensation-blocking agent.

11. The composition according to claim 4 wherein the amount of amine-containing compound ranges from about 0.75 percent to about 10 percent by weight of the composition and the amount of the potassium salt is effective to provide potassium ion in the composition at a range from about 0.15 percent to about 10 percent by weight of the composition.

12. The composition according to claim 4 wherein the amount of amine-containing compound ranges from about 0.75 percent to about 2 percent by weight of the composition and the amount of potassium salt is effective to provide potassium ion in the composition at a range from about 0.75 percent to about 2 percent by weight of the composition.

13. The composition according to claim 7 wherein the amount of amine-containing compound ranges from about 0.75 percent to about 10 percent by weight of the composition and the amount of the potassium channel agonist ranges from about 0.25 percent to about 10 percent by weight of the composition.

14. The composition according to any one of claims 1 to 13 wherein the composition provides at least about 25 percent inhibition of ear edema.

15. The composition according to any one of claims 1 to 13 wherein the composition provides at least about 50 percent inhibition of scratching.

## Patentansprüche

1. Zusammensetzung, umfassend:
eine aminhaltige Verbindung, ausgewählt aus der Gruppe bestehend aus Formel I und Formel II, 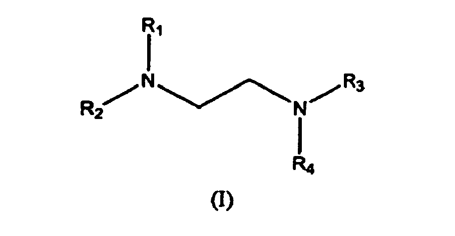 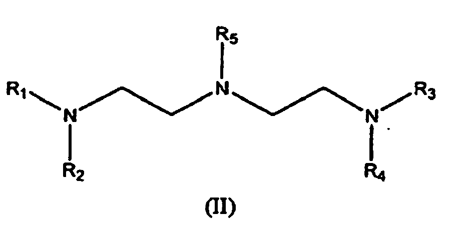 in denen R₁, R₂, R₃, R₄ und R₅ unabhängig aus der Gruppe bestehend aus Wasserstoff, C₁-C₆-Alkyl und C₁-C₆-Hydroxyalkyl ausgewählt sind; oder ein kosmetisch unbedenkliches Salz davon; und
ein empfindungsblockierendes Mittel, ausgewählt aus der Gruppe bestehend aus einem Kaliumsalz und einem Kaliumkanalagonisten,
wobei die aminhaltige Verbindung und das empfindungsblockierende Mittel in Mengen vorliegen, die ausreichen, um beim Auftragen auf die Haut eine schmerzstillende Wirkung zu erzielen.

2. Zusammensetzung nach Anspruch 1, wobei die aminhaltige Verbindung aus der Gruppe bestehend aus N,N,N',N'-Tetrakis(2-hydroxypropyl)ethylendiamin und Tris[2-(isopropylamino)ethyl]amin ausgewählt ist.

3. Zusammensetzung nach Anspruch 1 oder Anspruch 2, wobei die Menge an aminhaltiger Verbindung im Bereich von ungefähr 0,75 Gew.-% bis ungefähr 10 Gew.-% der Zusammensetzung, vorzugsweise im Bereich von ungefähr 0,75 Gew.-% bis ungefähr 2 Gew.-% der Zusammensetzung, liegt.

4. Zusammensetzung nach einem der Ansprüche 1 bis 3, wobei es sich bei dem empfindungsblockierenden Mittel um ein Kaliumsalz handelt.

5. Zusammensetzung nach Anspruch 4, wobei das Kaliumsalz aus der Gruppe bestehend aus Kaliumlactat, Kaliumbromid, Kaliumcarbonat, Kaliumacetat, Kaliumchlorat, Kaliumchlorid, Kaliumchromat, Kaliumcyanid, Kaliumdichromat, Kaliumiodid, Kaliumnitrat, Kaliumsulfat, Kaliumpyrophosphat, Kaliumfluorosilicat und Kaliumnatriumtartrat ausgewählt ist.

6. Zusammensetzung nach Anspruch 4 oder Anspruch 5, wobei die Menge des Kaliumsalzes ausreicht, um Kaliumionen in der Zusammensetzung in einem Bereich von ungefähr 0,15 Gew.-% bis ungefähr 10 Gew.-% der Zusammensetzung, vorzugsweise von ungefähr 0,20 Gew.-% bis ungefähr 2 Gew.-% der Zusammensetzung, bereitzustellen.

7. Zusammensetzung nach einem der Ansprüche 1 bis 3, wobei es sich bei dem empfindungsblockierenden Mittel um einen Kaliumkanalagonisten handelt.

8. Zusammensetzung nach Anspruch 7, wobei der Kaliumkanalagonist aus der Gruppe bestehend aus Minoxidil, Pinacidil, Diazoxid, Cromakalin, Nicorandil und Aprikalim ausgewählt ist.

9. Zusammensetzung nach Anspruch 7 oder Anspruch 8, wobei die Menge an Kaliumkanalagonisten im Bereich von ungefähr 0,25 Gew.-% bis ungefähr 10 Gew.-% der Zusammensetzung, vorzugsweise von ungefähr 0,25 Gew.-% bis ungefähr 2 Gew.-% der Zusammensetzung, liegt.

10. Zusammensetzung nach Anspruch 1 bis 9, umfassend ungefähr 0,75 Gew.-% bis ungefähr 10 Gew.-% der aminhaltigen Verbindung und ungefähr 0,25 Gew.-% bis ungefähr 10 Gew.-% des empfindungsblockierenden Mittels.

11. Zusammensetzung nach Anspruch 4, wobei die Menge an aminhaltiger Verbindung im Bereich von ungefähr 0,75 Gew.-% bis ungefähr 10 Gew.-% der Zusammensetzung liegt und die Menge an Kaliumsalz ausreicht, um Kaliumionen in der Zusammensetzung in einem Bereich von ungefähr 0,15 Gew.-% bis ungefähr 10 Gew.-% der Zusammensetzung bereitzustellen.

12. Zusammensetzung nach Anspruch 4, wobei die Menge an aminhaltiger Verbindung im Bereich von ungefähr 0,75 Gew.-% bis ungefähr 2 Gew.-% der Zusammensetzung liegt und die Menge an Kaliumsalz ausreicht, um Kaliumionen in der Zusammensetzung in einem Bereich von ungefähr 0,75 Gew.-% bis ungefähr 2 Gew.-% der Zusammensetzung bereitzustellen.

13. Zusammensetzung nach Anspruch 7, wobei die Menge an aminhaltiger Verbindung im Bereich von ungefähr 0,75 Gew.-% bis ungefähr 10 Gew.-% der Zusammensetzung liegt und die Menge an Kaliumkanalagonisten im Bereich von 0,25 Gew.-% bis ungefähr 10 Gew.-% der Zusammensetzung liegt.

14. Zusammensetzung nach einem der Ansprüche 1 bis 13, wobei die Zusammensetzung bei Ohrödem eine Hemmung von mindestens ungefähr 25% bewirkt.

15. Zusammensetzung nach einem der Ansprüche 1 bis 13, wobei die Zusammensetzung bei Juckreiz eine Hemmung von mindestens ungefähr 50% bewirkt.

## Revendications

1. Composition, comprenant :
un composé contenant une amine choisi dans le groupe constitué de la formule I et la formule II 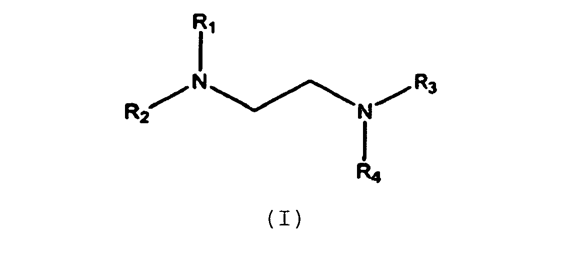 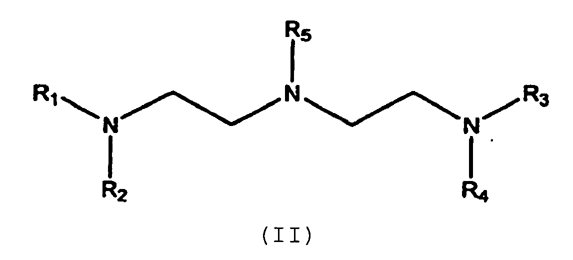 dans lesquelles R₁, R₂, R₃, R₄ et R₅ sont indépendamment choisis dans le groupe constitué d'un hydrogène, un alkyle en C₁-C₆, et un hydroxyalkyle en C₁-C₆ ; ou un sel acceptable en cosmétique de celui-ci ; et
un agent bloquant la sensation choisi dans le groupe constitué d'un sel de potassium et d'un agoniste de canal potassique,
le composé contenant une amine et l'agent bloquant la sensation étant présents dans des quantités efficaces pour obtenir un effet analgésique sur la peau lorsqu'elle est appliquée sur celle-ci.

2. Composition selon la revendication 1 dans laquelle le composé contenant une amine est choisi dans le groupe constitué de la N,N,N',N'-tétrakis(2-hydroxypropyl)éthylènediamine et la tris[2-(isopropylamino)éthyl]amine.

3. Composition selon la revendication 1 ou la revendication 2 dans laquelle la quantité de composé contenant une amine est dans la plage d'environ 0,75 pour cent à environ 10 pour cent en poids de la composition, de préférence d'environ 0,75 pour cent à environ 2 pour cent en poids de la composition.

4. Composition selon l'une quelconque des revendications 1 à 3 dans laquelle l'agent bloquant la sensation est un sel de potassium.

5. Composition selon la revendication 4 dans laquelle le sel de potassium est choisi dans le groupe constitué des lactate de potassium, bromure de potassium, carbonate de potassium, acétate de potassium, chlorate de potassium, chlorure de potassium, chromate de potassium, cyanure de potassium, dichromate de potassium, iodure de potassium, nitrate de potassium, sulfate de potassium, pyrophosphate de potassium, fluorosilicate de potassium et tartrate de potassium-sodium.

6. Composition selon la revendication 4 ou la revendication 5 dans laquelle la quantité du sel de potassium est efficace pour fournir des ions potassium dans la composition dans une plage d'environ 0,15 pour cent à environ 10 pour cent en poids de la composition, de préférence d'environ 0,20 pour cent à environ 2 pour cent en poids de la composition.

7. Composition selon l'une quelconque des revendications 1 à 3 dans laquelle l'agent bloquant la sensation est un agoniste de canal potassique.

8. Composition selon la revendication 7 dans laquelle l'agoniste de canal potassique est choisi dans le groupe constitué des minoxidil, pinacidil, diazoxide, cromakaline, nicorandil et aprilakim.

9. Composition selon la revendication 7 ou la revendication 8 dans laquelle la quantité de l'agoniste de canal potassique est dans la plage d'environ 0,25 pour cent à environ 10 pour cent en poids de la composition, de préférence d'environ 0,25 pour cent à environ 2 pour cent en poids de la composition.

10. Composition selon l'une quelconque des revendications 1 à 9 comprenant d'environ 0,75 pour cent à environ 10 pour cent en poids du composé contenant une amine et d'environ 0,25 pour cent à environ 10 pour cent en poids de l'agent bloquant la sensation.

11. Composition selon la revendication 4 dans laquelle la quantité de composé contenant une amine est dans la plage d'environ 0,75 pour cent à environ 10 pour cent en poids de la composition et la quantité du sel de potassium est efficace pour fournir des ions potassium dans la composition dans une plage d'environ 0,15 pour cent à environ 10 pour cent en poids de la composition.

12. Composition selon la revendication 4 dans laquelle la quantité de composé contenant une amine est dans la plage d'environ 0,75 pour cent à environ 2 pour cent en poids de la composition et la quantité de sel de potassium est efficace pour fournir des ions potassium dans la composition dans une plage d'environ 0,75 pour cent à environ 2 pour cent en poids de la composition.

13. Composition selon la revendication 7 dans laquelle la quantité de composé contenant une amine est dans la plage d'environ 0,75 pour cent à environ 10 pour cent en poids de la composition et la quantité de l'agoniste de canal potassique est dans la plage d'environ 0,25 pour cent à environ 10 pour cent en poids de la composition.

14. Composition selon l'une quelconque des revendications 1 à 13 dans laquelle la composition produit au moins environ 25 pour cent d'inhibition de l'oedème auriculaire.

15. Composition selon l'une quelconque des revendications 1 à 13 dans laquelle la composition produit au moins environ 50 pour cent d'inhibition des grattements.
